# EUROPEAN PATENT APPLICATION

(11) **EP 0 657 442 A2**
(43) Date of publication of application: **14.06.1995**
(21) Application number: 94118993.8
(22) Date of filing: 01.12.1994
(51) Int. Cl.: C07D 277/22, C07B 43/04

(54) **Thiazole derivate and process for its preparation**

(30) Priority: 02.12.1993 JP 302493/93
(71) Applicant: ASAHI CHEMICAL CO., LTD., Yodogawa-ku, Osaka-shi, Osaka 532 (JP)
(72) Inventor: Kamimura, Yasuo, Asahikagakukogyo, Mikuni-cho, Sakai-gun, Fukui-ken (JP); Kamiyama, Morihiro, Asahikagakukogyo, Mikuni-cho, Sakai-gun, Fukui-ken (JP)
(74) Representative: VOSSIUS & PARTNER

(57) **Abstract**

The invention relates to a thiazole derivative represented by the formula (1) 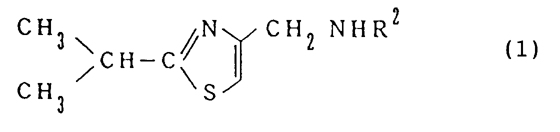
wherein R² is an alkyl group having 1 to 4 carbon atoms.

The thiazole derivative of the invention is prepared by reacting 2-isopropyl-4-chloromethylthiazole with an N-alkyl-acid amide-alkali metal salt in an aromatic hydrocarbon to give a thiazole-N-alkyl-acid amidated product, and hydrolyzing the resulting compound in the presence of an aromatic hydrocarbon.

The thiazole derivative of the formula (1) prepared according to the invention can be suitably used as information display materials, antiglare materials, and intermediates for preparation of medicaments, agricultural chemicals.

## Description

The present invention relates to a thiazole derivative and a process for preparing the derivative.

Various processes have been heretofore reported for preparing 2-isopropyl-4-chloromethylthiazole represented by the formula 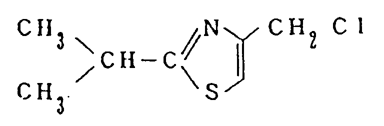
For example, Japanese Unexamined Publications Nos. 151183/1977 and 103168/1981 disclose the preparation of 2-isopropyl-4-chloromethylthiazole by reacting isobutyrothioamide with 1,3-dichloroacetone in ethanol. Agricultural and Biological Chemistry, vol. 36, No.13, pp. 2287 (1972) discloses the preparation of 2-isopropyl-4-chloromethylthiazole by reacting isobutyrothioamide with 1,3-dichloroacetone in benzene. However, these processes produce the objective 2-isopropyl-4-chloromethylthiazole merely in a low yield and with a low purity. Further the former process provides the objective compound in the form of a salt of hydrochloric acid, and thus essentially involves use of hydrogen chloride gas. On the other hand, the latter process employs sodium hydroxide to isolate the objective compound, inevitably resulting in the decomposition of the objective compound due to the sodium hydroxide used and thus in a pronounced decrease of yield and purity. In short, known processes for the preparation of 2-isopropyl-4-chloromethylthiazole are not commercially beneficial.

Thus, it is the object of the present invention to provide a thiazole derivative which can be suitably used as information display materials, antiglare materials, and intermediates for the preparation of medicaments, agricultural chemicals and the like, and to provide a commercially beneficial process for preparing said thiazole derivative.

This object has been achieved by a thiazole derivative represented by the formula (1) 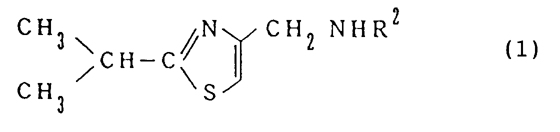
wherein R² is an alkyl group having 1 to 4 carbon atoms and by a process for preparing the derivative as shown below.

It is common practice to react a monoalkyl amine with 2-isopropyl-4-chloromethylthiazole for the substitution of N-alkylamino group for the chloro group of the thiazole. This method requires a monoalkyl amine in large excess and produces a tertiary amine and quaternary ammonium salt, which reduce the yield of the objective product and decrease the purity thereof due to their presence as contaminants. Consequently, the method can never be commercially advantageous.
However, the thiazole derivative of the formula (1) in accordance with the invention can be prepared by reacting 2-isopropyl-4-chloromethylthiazole represented by the formula (2) 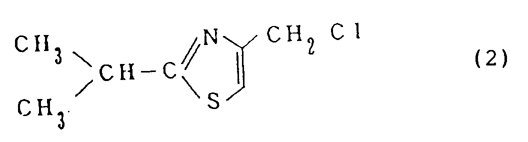
with an N-alkyl-acid amide-alkali metal salt represented by the formula (3) 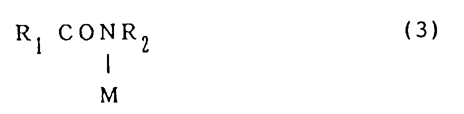
wherein R¹ is a hydrogen atom or a methyl group, R² is an alkyl group of 1 to 4 carbon atoms, and M is an alkali metal in an aromatic hydrocarbon to give a thiazole-N-alkyl-acid amidated product represented by the formula (4) 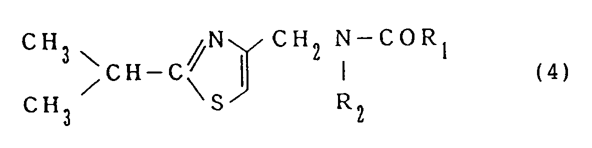
wherein R¹ and R² are as defined above, and hydrolyzing the compound of the formula (4) in the presence of an aromatic hydrocarbon.

The 2-isopropyl-4-chloromethylthiazole of the formula (2) to be used as one of the starting materials in the invention can be prepared by reacting isobutyrothioamide with 1,3-dichloroacetone in acetone. The proportions of isobutyrothioamide and 1,3-dichloroacetone used are not specifically limited, but it is suitable to use 1 to 1.3 moles, preferably 1.1 to 1.2 moles, of the former per mole of the latter. The reaction proceeds at a temperature of 0 to 50°C, preferably 20 to 30°C and is completed in about 8 to about 15 hours. According to the invention, preferably the compound prepared by the reaction of isobutyrothioamide and 1,3-dichloroacetone is neutralized with a carbonate or bicarbonate of an alkali metal, and the produced 2-isopropyl-4-chloromethylthiazole is isolated by distillation.

The N-alkyl-acid amide-alkali metal salt of the formula (3) to be used as the other starting material in the invention can be prepared, for example, by reacting N-alkyl-acid amide of the formula R¹CONHR² (wherein R¹ and R² are as defined above) with an alkali metal of the formula M (wherein M is as defined above) or an alkali metal amide of the formula MNH₂ (wherein M is as defined above). The proportions of N-alkyl-acid amide and alkali metal or alkali metal amide used are not specifically limited, but it is suitable to use 1 to 5 moles, preferably 1.5 to 3 moles, of the former per mole of the latter. The reaction is conducted usually in a suitable solvent. Useful solvents include, for example, benzene, toluene, xylene, naphthalene, kerosine, hexane, etc. The reaction proceeds at a temperature in the range of 40 to 120°C, preferably 60 to 100°C and is completed in about 1 to about 3 hours.

The reaction between the 2-isopropyl-4-chloromethylthiazole of the formula (2) and the N-alkyl-acid amide-alkali metal salt of the formula (3) is effected in an aromatic hydrocarbon. Useful aromatic hydrocarbons include a wide range of those heretofore known such as benzene, toluene, xylene, mixtures thereof, etc. Among them, toluene and xylene are suitable to use. The proportions of 2-isopropyl-4-chloromethylthiazole of the formula (2) and N-alkyl-acid amide-alkali metal salt of the formula (3) used are not essential, but it is suitable to use 1 to 3 moles, preferably 1.1 to 2.5 moles, of the latter per mole of the former. The reaction proceeds at a temperature in the range of 40 to 120°C, preferably 50 to 100°C and is completed in about 2 to about 3 hours.

In the practice of the invention, the thiazole-N-alkyl-acid amidated product of the formula (4) produced by the reaction may be isolated from the reaction mixture, or alternatively the reaction mixture may be subjected to the subsequent reaction without isolation.

The hydrolysis of the compound of the formula (4) is performed in the presence of an aromatic hydrocarbon. Useful aromatic hydrocarbons include a wide range of those heretofore known such as benzene, toluene, xylene, mixtures thereof, etc. Among them, toluene and xylene are preferred. The hydrolysis can be carried out in the conventional manner, preferably in the presence of a mineral acid. Useful mineral acids are, for example, hydrochloric acid, sulfuric acid, etc. The reaction proceeds at a temperature in the range of 50 to 90°C, preferably 60 to 80°C and is completed in about 2 to about 3 hours.

The compound of the present invention prepared by the reaction can be easily isolated from the reaction mixture for purification.

According to the process of the present invention, the desired thiazole derivative of the formula (1) can be produced by a simple procedure in a high yield and with a high purity.

Given below is a listing of the results attainable by carrying out preferred embodiments of the process of the invention.
(a) Isobutyrothioamide is reacted with 1,3-dichloroacetone in acetone, and the reaction mixture is neutralized with a carbonate or bicarbonate of an alkali metal, whereby 2-isopropyl-4-chloromethylthiazole of the formula (2) can be isolated by distillation from the reaction mixture without change of properties in a high yield and with a high purity.
(b) The 2-isopropyl-4-chloromehylthiazole of the formula (2) is reacted with the N-alkyl-acid amide-alkali metal salt of the formula (3) to accomplish the substitution of N-alkylamino group for the chloro group, giving a thiazole-N-alkyl-acid amidated product of the formula (4), whereby the selectivity and conversion of the objective compound of the formula (1) are increased.
(c) When a mineral acid is used in the hydrolysis of the thiazole-N-alkyl-acid amidated product of the formula (4), the reaction is completed in a shorter time and the objective compound of the formula (1) can be produced with a higher purity.
(d) When an aromatic hydrocarbon is used as a solvent both in the reaction between the 2-isopropyl-4-chloromethylthiazole of the formula (2) and the N-alkyl-acid amide-alkali metal salt of the formula (3) and in the hydrolysis of the thiazole-N-alkyl-acid amidated product of the formula (4), the reactions can be performed in one pot. Further, the aromatic hydrocarbon can be used as a solvent for extraction after the completion of the reaction.
(e) A high-purity objective compound of the formula (1) can be isolated from the reaction mixture by a simple procedure of distilling the extract obtained using the aromatic hydrocarbon as a solvent.
(f) The objective compounds having various alkyl groups can be produced by alteration of alkyl groups of N-alkyl-acid amide.

The thiazole derivative of the formula (1) prepared according to the invention can be suitably used as information display materials, antiglare materials, and intermediates for preparation of medicaments, agricultural chemicals and the like. Especially the thiazole derivative of the formula (1) has a thermochromic characteristic which is utilizable for information display materials and antiglare materials.

The present invention will be described below in more detail with reference to the following reference examples and examples.

### Reference Example 1

A 500 ml, 4-necked flask equipped with a stirrer, dropping funnel and thermometer was charged with 56.75 g (0.55 mole) of isobutyrothioamide and 125 ml of acetone to obtain a solution. An acetone solution (126.97 g) containing 63.49 g (0.50 mole) of 1,3-dichloroacetone was added dropwise with stirring while maintaining the mixture at 24 to 26°C. After completion of the addition, the stirring was continued over night to complete the reaction. After filtering off a small amount of insolubles, the acetone filtrate was concentrated under reduced pressure. Into the concentrated residue, 250 ml of water and 100 ml of toluene were poured. Then, 48.00 g (0.57 mole) of sodium bicarbonate was added in small amounts and the mixture was neutralized. After the separation of toluene layer, the residue was subjected to vacuum distillation, giving 74.66 g of 2-isopropyl-4-chloromethylthiazole. The purity of the product was 97.2% and the yield was 85%.

### Reference Example 2

The procedure of Reference Example 1 was repeated with the exception of using 30.21 g (0.29 mole) of sodium carbonate in place of sodium bicarbonate. Vacuum distillation gave 72.91 g of 2-isopropyl-4-chloromethylthiazole. The purity of the product was 98.0% and the yield was 83%.

### Example 1

A 1,000 ml, 4-necked flask equipped with a stirrer, dropping funnel and thermometer was charged with 39.02 g (1.00 mole) of sodium amide and 300 ml of toluene. The charge was stirred to obtain a dispersion. The dispersion was heated to 90°C and 88.61 g (1.50 moles) of N-methylformamide was added dropwise, giving N-methylformamide-sodium salt. A toluene solution (135 g) containing 70.27 g (0.40 mole) of 2-isopropyl-4-chloromethylthiazole was added dropwise while maintaining the mixture at 95°C. After completion of the addition, the mixture was stirred for 2 hours at the same temperature and cooled to about 20°C. Into the reaction mixture was poured 250 ml of water to dissolve the salt in water. After the aqueous layer present as a lower one was separated, 219 g of 20% hydrochloric acid was poured into the toluene layer and the mixture was stirred at 70°C for 3 hours to hydrolyze a thiazole-N-methylformamide. After completion of the reaction, the reaction mixture was cooled to about 20°C. The aqueous layer was rendered strongly alkaline by addition of 200 g (1.60 moles) of 32% sodium hydroxide, and the separated oil was extracted with the toluene in the system. After separation of the toluene layer, vacuum distillation was effected, giving 55.17 g of the objective compound, i.e. 2-isopropyl-4-(N-methyl)aminomethylthiazole. The purity of the product was 99.8% and the yield was 81%.
Pale yellow transparent liquid
Boiling point: 83°C/3 torr
Infrared absorption spectrum: 3270, 3090, 2850, 1650, 1445, 1120 cm⁻¹

### Example 2

The procedure of Example 1 was repeated with the exception of using 22.99 g (1.00 mole) of a metallic sodium in place of sodium amide. The procedure gave 51.08 g of 2-isopropyl-4-(N-methyl)aminomethylthiazole. The purity of the product was 99.8% and the yield was 75%.

### Example 3

The procedure of Example 2 was repeated with the exception of changing the amounts of metallic sodium and N-methylformamide to 10.12 g (0.44 mole) and 70.88 g (1.20 moles), respectively. The procedure gave 49.04 g of 2-isopropyl-4-(N-methyl)aminomethylthiazole. The purity of the product was 99.8% and the yield was 72%.

### Example 4

The procedure of Example 3 was repeated with the exception of using a toluene solution containing 87.72 g (1.20 moles) of N-methylacetoamide in place of N-methylformamide, and 196 g (0.60 mole) of 30% sulfuric acid in place of 20% hydrochloric acid. The procedure gave 40.08 g of 2-isopropyl-4-(N-methyl)aminomethylthiazole. The purity of the product was 99.8% and the yield was 59%.

### Example 5

The procedure of Example 3 was repeated with the exception of using xylene in place of toluene, giving 42.23 g of 2-isopropyl-4-(N-methyl)aminomethylthiazole. The purity of the product was 99.8% and the yield was 62%.

## Claims

1. A thiazole derivative represented by the formula (1) 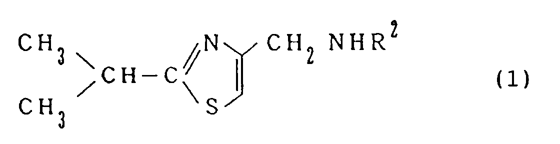 wherein R² is an alkyl group having 1 to 4 carbon atoms.

2. A process for preparing a thiazole derivative of the formula (1) 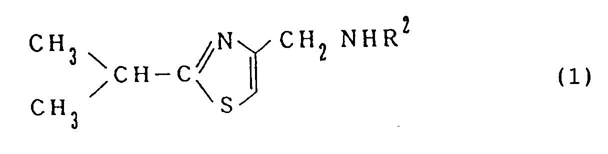 wherein R² is an alkyl group having 1 to 4 carbon atoms, the process comprising the steps of reacting 2-isopropyl-4-chloromethylthiazole represented by the formula (2) 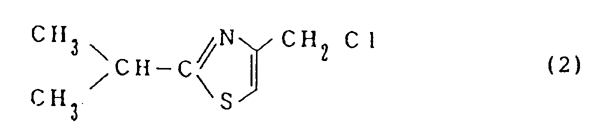 with an N-alkyl-acid amide-alkali metal salt represented by the formula (3) 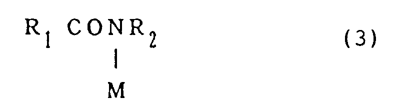 wherein R¹ is a hydrogen atom or a methyl group, R² is an alkyl group of 1 to 4 carbon atoms, and M is an alkali metal in an aromatic hydrocarbon to give a thiazole-N-alkyl-acid amidated product represented by the formula (4) 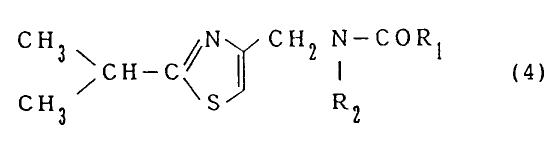 wherein R¹ and R² are as defined above, and hydrolyzing the compound of the formula (4) in the presence of an aromatic hydrocarbon.

3. A process according to claim 2 wherein the 2-isopropyl-4-chloromethylthiazole of the formula (2) is a compound prepared by reacting isobutyrothioamide with 1,3-dichloroacetone in acetone.

4. A process according to claim 3 wherein the compound prepared by the reaction of isobutyrothioamide and 1,3-dichloroacetone is neutralized with a carbonate or bicarbonate of an alkali metal, and the produced 2-isopropyl-4-chloromethylthiazole is isolated by distillation.

5. A process according to any of claims 2 to 4 wherein the N-alkyl-acid amide-alkali metal salt is a compound prepared by reacting N-alkyl-acid amide of the formula R¹CONHR² (wherein R¹ and R² are as defined above) with an alkali metal of the formula M (wherein M is as defined above) or an alkali metal amide of the formula MNH₂ (wherein M is as defined above).

6. A process according to any of claims 2 to 5 wherein the hydrolysis is conducted using a mineral acid.

7. A process according to any of claims 2 to 6 wherein the aromatic hydrocarbon is toluene and/or xylene.

8. Use of the thiazole derivative as defined in claim 1 as information display materials.

9. Use of the thiazole derivative as defined in claim 1 as antiglare materials.

10. Use of the thiazole derivative as defined in claim 1 as intermediates for the preparation of medicaments and/or agricultural chemicals.
